# EUROPEAN PATENT SPECIFICATION

(11) **EP 2 956 773 B1**
(45) Date of publication and mention of the grant of the patent: **21.10.2020**
(21) Application number: 14752049.8
(22) Date of filing: 11.02.2014
(51) Int. Cl.: G01N 33/574, G01N 33/58

(54) **METHOD FOR DETECTING SHED OR CIRCULATING TUMOR CELLS IN BIOLOGICAL FLUIDS**
VERFAHREN ZUR ERKENNUNG VON SHED- ODER ZIRKULIERENDEN TUMORZELLEN IN BIOLOGISCHEN FLÜSSIGKEITEN
PROCÉDÉ DE DÉTECTION DE CELLULES TUMORALES DÉVERSÉES OU EN CIRCULATION DANS DES LIQUIDES BIOLOGIQUES

(30) Priority: 15.02.2013 US 201361765329 P
(43) Date of publication of application: 23.12.2015
(73) Proprietor: Thomas Jefferson University, Philadelphia, PA 19107 (US)
(72) Inventor: THAKUR, Mathew, L., Cherry Hill, NJ 08003 (US); GOMELLA, Leonard, G., Chadds Ford, PA 19317 (US)
(74) Representative: Forstmeyer, Dietmar
(86) International application number: PCT/US2014/015758
(87) International publication number: WO 2014/126904

(56) References cited:
- WO-A2-2011/054001
- US-A1- 2010 297 634
- US-A1- 2010 297 634
- US-B1- 6 608 174
- VIRGOLINI I ET AL: "I-Vasoactive Intestinal Peptide (VIP) Receptor Scanning: Update of Imaging Results in Patients with Adenocarcinomas and Endocrine Tumors of the Gastrointestinal Tract", NUCLEAR MEDICINE AND BIOLOGY, ELSEVIER, NY, US, vol. 23, no. 6, 1 August 1996 (1996-08-01) , pages 685-692, XP004070331, ISSN: 0969-8051, DOI: 10.1016/0969-8051(96)00066-2
- ZHANG ET AL: "Vasoactive intestinal peptide (VIP) and pituitary adenylate cyclase activating peptide (PACAP) receptor specific peptide analogues for PET imaging of breast cancer: In vitro/in vivo evaluation", REGULATORY PEPTIDES, ELSEVIER SCIENCE BV, NL, vol. 144, no. 1-3, 6 November 2007 (2007-11-06), pages 91-100, XP022331940, ISSN: 0167-0115, DOI: 10.1016/J.REGPEP.2007.06.008
- REUBI JEAN CLAUDE ET AL: "Vasoactive intestinal peptide/pituitary adenylate cyclase-activating peptide receptor subtypes in human tumors and their tissues of origin", CANCER RESEARCH, AMERICAN ASSOCIATION FOR CANCER RESEARCH, US, vol. 60, no. 11, 1 June 2000 (2000-06-01), pages 3105-3112, XP002427944, ISSN: 0008-5472
- REUBI J C: "IN VITRO IDENTIFICATION OF VASOACTIVE INTESTINAL PEPTIDE RECEPTORS IN HUMAN TUMORS: IMPLICATIONS FOR TUMOR IMAGING", THE JOURNAL OF NUCLEAR MEDICINE, SOCIETY OF NUCLEAR MEDICINE, US, vol. 36, no. 10, 1 October 1995 (1995-10-01), pages 1846-1853, XP001150723, ISSN: 0161-5505
- M GARCIA FERNANDEZ ET AL: "Expression of functional PACAP/VIP receptors in human prostate cancer and healthy tissue", PEPTIDES, vol. 24, no. 6, 1 June 2003 (2003-06-01), pages 893-902, XP055235631, AMSTERDAM, NL ISSN: 0196-9781, DOI: 10.1016/S0196-9781(03)00162-1
- GLENN PAUL DORSAM: "Vasoactive intestinal peptide signaling axis in human leukemia", THE JOURNAL OF BIOLOGICAL CHEMISTRY, vol. 2, no. 6, 1 January 2011 (2011-01-01) , page 146, XP055309570, ISSN: 1949-8454, DOI: 10.4331/wjbc.v2.i6.146
- THAKUR: 'Genomic Biomarkers for Molecular Imaging: Predicting the Future' SEMIN NUCL MED vol. 39, no. 4, July 2009, pages 236 - 246, XP026162493
- ZHANG ET AL.: 'Vasoactive intestinal peptide (VIP) and pituitary adenylate cyclase activating peptide (PACAP) receptor specific peptide analogues for PET imaging of breast cancer: In vitro/in vivo evaluation.' REGUL PEPT vol. 144, no. 1-3, 04 December 2007, pages 91 - 100, XP022331940
- THAKUR ET AL.: 'PET imaging of oncogene overexpression using 64Cu-vasoactive intestinal peptide (VIP) analog: comparison with 99mTc-VIP analog.' J NUCL MED vol. 45, no. 8, August 2004, pages 1381 - 1389, XP055235624
- GARCIA-FERNANDEZ ET AL.: 'Expression of functional PACAPNIP receptors in human prostate cancer and healthy tissue.' PEPTIDES vol. 24, no. 6, June 2003, pages 893 - 902, XP055235631
- X. Wang ET AL: "Detection of Circulating Tumor Cells in Human Peripheral Blood Using Surface-Enhanced Raman Scattering Nanoparticles", CANCER RESEARCH, vol. 71, no. 5, 6 January 2011 (2011-01-06), pages 1526-1532, XP055417410, US ISSN: 0008-5472, DOI: 10.1158/0008-5472.CAN-10-3069
- GREENBAUM D ET AL: "COMPARING PROTEIN ABUNDANCE AND MRNA EXPRESSION LEVELS ON A GENOMIC SCALE", GENOME BIOLOGY (ONL, BIOMED CENTRAL LTD, GB, vol. 40, no. 9, 1 January 2003 (2003-01-01), pages 117.01-117.08, XP008036618, ISSN: 1465-6914
- Elizabeth A. Punnoose ET AL: "Molecular Biomarker Analyses Using Circulating Tumor Cells", PLoS ONE, vol. 5, no. 9, 1 January 2010 (2010-01-01) , page e12517, XP055031075, ISSN: 1932-6203, DOI: 10.1371/journal.pone.0012517
- D. C. Danila ET AL: "Circulating Tumor Cells as Biomarkers in Prostate Cancer", CLINICAL CANCER RESEARCH, vol. 17, no. 12, 15 June 2011 (2011-06-15) , pages 3903-3912, XP055256648, US ISSN: 1078-0432, DOI: 10.1158/1078-0432.CCR-10-2650

## Description

### Background of the Invention

There is evidence that primary cancers begin shedding neoplastic cells into the circulation at an early disease stage prior to the appearance of clinical manifestations. Malignant tumors are characterized by their ability to invade adjacent tissue. In general, tumors with a diameter of 1 mm are vascularized and animal studies show that as much as 4% of the cells present in the tumor can be shed into the circulation in a 24 hour period (Butler & Gullino (1975) Cancer Res. 35:512-516).

Various methods for isolating and detecting tumor cells in blood have been described. For example, US 6,190,870 teaches immunomagnetic isolation followed by flow cytometric enumeration. However, there is also no visual analysis of the samples. US 6,197,523 describes enumerating cancer cells in 100 µl blood samples. The methods use capillary microscopy to confirm the identity of cells that are found. Further, US 6,365,362 describes methods for immunomagnetically enriching and analyzing samples for tumor cells in blood. In accordance with the methods of this patent, the isolated cells are labeled for the presence of nucleic acid and an additional marker, which allows the exclusion of non-target sample components during analysis. WO 02/20825 describes using an adhesion matrix for enumerating tumor cells. Briefly, the matrix is coated with specific adhesion molecules that will bind to cancer cells with metastatic potential. The matrix can then be analyzed for the presence and type of captured cells.

Recent approaches to drug discovery focus on understanding the genesis of disease and the biomedical pathways controlling disease at a molecular level. Previous studies have demonstrated that VPAC receptors (combined for vasoactive intestinal peptide (VIP) and pituitary adenylate cyclase activating peptide (PACAP)) are overexpressed in high density on BC cells (Reubi, et al. (2000) Cancer Res. 60(11):3105-3112). VPAC receptors are involved in cell proliferation, cell differentiation, as well as in survival of BC cells. On stroma, normal cells, and benign masses only a few VPAC receptors are expressed (Reubi, et al. (2000) *supra;* Zia, et al. (1996) Cancer Res. 56(15) :3486-89; Leyton, et al. (1999) Breast Canc. Res. Treat. 56(2) :177-186; Moody & Gozes I (2007) Curr. Pharm. Des. 13(11):1099-1104; Valdehita, et al. (2010) Peptides 31 (11) :2035-2045; Valdehita, et al. (2012) Mol. Cell Endocrinol. 348(1) :241-246) .

Radiolabeled biomolecules with high affinities for VPAC receptors have been developed and analyzed in a preclinical setting (Chakder & Rattan (1993) J. Pharm. Expt. Therapeut. 266:392-399; Thakur, et al. (2000) J. Nucl. Med. 41:107-110; Pallela, et al. (1999) J. Nucl. Med. 40(2):352-360; Kolan, et al. (1997) J. Label. Comp. Radiopharmaceut. 40:455-457; Thakur, et al. (2004) J. Nucl. Med. 45:1381-1389; Zhang, et al. (2007) Reg. Peptides 144:91-100; Thakur (2009) Semin. Nucl. Med. 39:236-246; Thakur, et al. (2010) J. Nucl. Med. 51:106-111; Zhang, et al. (2008) J. Nucl. Med. 49:112-121; US 6,855,308). Based on their high affinity for VPAC receptors, peptide constructs, modified by radiolabeling with Tc99m (t½ - 6 hours, γ - 140 keV), have been generated and evaluated for receptor affinity (Kd), receptor specificity, *in vivo* stability and tissue distribution (Thakur, et al. (2000) *supra;* Pallela, et al. (1999) *supra;* Kolan, et al. (1997) *supra*; Thakur, et al. (2004) *supra;* Zhang, et al. (2007) supra; Thakur (2009) *supra;* Thakur, et al. (2010) *supra;* Zhang, et al. (2008) *supra*). In addition, peptides have been labeled with β+ (19%, 656 keV) emitting Cu-64 (t½ - 12.8 hours) for positron emission tomography (PET) with N₂S₂ as a chelating agent. Kd values, tissue distribution studies in athymic nude mice bearing T47D human BC, receptor blocking studies, receptor affinity, and *in vivo* stability have been examined (Thakur, et al. (2004) *supra;* Zhang, et al. (2007) *supra;* Thakur (2009) *supra;* Thakur, et al. (2010) *supra;* Zhang, et al. (2008) *supra*). Of the compounds produced, Cu-64-TP3805 not only imaged all xenografted human BC in athymic nude mice (tumor uptake 6.35±1.28% ID/g at 24 hours post-injection), this compound also localized all (n=8), spontaneously grown BC (visible 5, invisible 1 and metastatic 2) lesions in transgenic MMTVneu mice (n=9) (Thakur, et al. (2010) *supra*). Furthermore, the Cu-64-TP3805 PET images were normal for two lesions that had negligible expression of VPAC1 receptors. The eight malignant lesions were confirmed by histology and expressed VPAC1 receptors as determined by RT-PCR (Thakur, et al. (2010) *supra).*

US 2010/297634 A1 discloses methods of identifying and distinguishing endothelial progenitor cells in the blood from circulating tumor cells including metastatic cells and, further, distinguishing among circulating tumor cells that originate from diverse tumors of epithelial lineage.

Wang et al., Cancer Res, 71(5), 1526-1532 (2011; doi:10.1158/0008-5472.CAN-10-3069) teach detection and characterization of circulating tumor cells in human peripheral blood using surface-enhanced Raman spectroscopy.

### Summary of the Invention

The present invention provides:
(1) A method for detecting shed or circulating tumor cells from prostate cancer comprising contacting a urine sample with a labeled pituitary adenylate cyclase activating peptide (PACAP) or vasoactive intestinal peptide (VIP), and determining binding of the PACAP or VIP to shed or circulating tumor cells from prostate cancer in the urine sample thereby detecting shed or circulating tumor cells from prostate cancer;
(2) the method of (1), wherein the label that is coupled to the PACAP or VIP peptide is or contains a chromophore, fluorophore or protein;
(3) a method for diagnosing prostate cancer comprising
   (a) contacting a urine sample with a labeled pituitary adenylate cyclase activating peptide (PACAP) or vasoactive intestinal peptide (VIP);
   (b) detecting binding of PACAP or VIP to shed or circulating tumor cells from prostate cancer in the urine sample;
   (c) comparing binding of PACAP or VIP to shed or circulating tumor cells from prostate cancer in the urine sample to PACAP or VIP binding in a control sample; and
   (d) diagnosing prostate cancer, wherein the presence of binding of PACAP or VIP to shed or circulating tumor cells from prostate cancer in the urine sample is indicative of the subject having cancer;
(4) the method of (3), wherein the label that is coupled to the PACAP or VIP peptide is or contains a chromophore, fluorophore or protein.

### Detailed Description of the Invention

The aspects of the invention are defined in the attached claims. It has now been found that a labeled pituitary adenylate cyclase activating peptide (PACAP) can bind the VCAP1 receptor and detect tumor cells from prostate cancer present in urine. The labeled peptide can detect cells at a concentration of 5 cells/ml sample and correctly identify and distinguish cancer cells from contaminating epithelial cells and white blood cells. The use of this labeled peptide in detecting tumor cells is simple, efficient and does not require expensive equipment or training.

Therefore, the present invention provides methods for detecting tumor cells from prostate cancer and diagnosing prostate cancer using labeled PACAPs or vasoactive intestinal peptides (VIP), both of which bind VPAC1 receptors. In accordance with the present invention, a PACAP is intended to mean a peptide having the sequence of His-Ser-Asp-Gly-Ile-Phe-Thr-Asp-Ser-Tyr-Ser-Arg-Tyr-Arg-Lys-Gln-Met-Ala-Val-Lys-Lys-Tyr-Leu-Ala-Ala-Val-Leu-Gly-Lys-Arg-Tyr-Lys-Gln-Arg-Val-Lys-Asn-Lys (SEQ ID NO:1), or a 29 to 37 amino acid residue fragment thereof. In certain embodiments, the 29 to 37 amino acid residue fragment includes the N-terminal 29 to 37 amino acid residues of the PACAP of SEQ ID NO:1. In particular embodiments, the PACAP has the sequence His-Ser-Asp-Gly-Ile-Phe-Thr-Asp-Ser-Tyr-Ser-Arg-Tyr-Arg-Lys-Gln-Met-Ala-Val-Lys-Lys-Tyr-Leu-Ala-Ala-Val-Leu (SEQ ID NO:2).

A VIP is intended to mean a peptide having the sequence of His-Ser-Asp-Ala-Val-Phe-Thr-Asp-Asn-Tyr-Thr-Arg-Leu-Arg-Lys-Gln-Met-Ala-Val-Lys-Lys-Tyr-Leu-Asn-Ser-Ile-Leu-Asn (SEQ ID NO:3), or derivatives thereof, *e*.*g*., His(Ac) -Ser-Asp-Ala-Val-Phe-Thr-Asp-Asn-Tyr-Thr-Arg-Leu-Arg-Lys-Gln-Met(O)-Ala-Val-Lys-Lys-Tyr-Leu-Asn-Ser-Ile-Leu-Asn (SEQ ID NO:4); His(Ac)-Ser-Asp-Ala-Val-Phe-Thr-Asp-Asn-Tyr-Thr-Arg-Leu-Arg-Lys-Gln-Met-Ala-Val-Lys-Lys-Tyr-Leu-Asn-Ser-Val-Leu-Asn (SEQ ID NO:5); His(Ac)-Ser-Asp-Ala-Val-Phe-Thr-Asp-Asn-Tyr-Thr-Arg-Leu-Arg-Lys-Gln-Met(O)-Ala-Val-Lys-Lys-Tyr-Leu-Asn-Ser-Val-Leu-Asn (SEQ ID NO:6), wherein His(Ac) means N-acetyl histidine, and Met(O) means methionine oxide and wherein said peptides have been shown to exhibit the biological activity of VIP (see US 8,329,640).

In some embodiments, one or two of the amino acid residues of the PACAP or VIP have been replaced with a nonstandard amino acid residue. In accordance with this embodiment, a nonstandard amino acid residue includes, but is not limited to, norvaline, norleucine, α-amino-n-butyric acid, α,γ-diaminobutyric acid, γ-aminobutyric acid, ornithine, isovaline, N-ethyl glycine, N-methyl alanine, and isoserine. In certain embodiments, the nonstandard amino acid residue is γ-aminobutyric acid. In particular embodiments, the PACAP has the sequence His-Ser-Asp-Gly-Ile-Phe-Thr-Asp-Ser-Tyr-Ser-Arg-Tyr-Arg-Lys-Gln-Met-Ala-Val-Lys-Lys-Tyr-Leu-Ala-Ala-Val-Leu-γAba-Lys (SEQ ID NO:7).

While peptides binding the VPAC1 receptor are particularly embraced by this invention, it is contemplated that shed or circulating tumor cells in biological fluids can also be detected using labeled peptides that bind to other markers. For example, binding to carcinoembryonic antigen (CEA) can be used to detect shed prostate or colorectal cancer; CD20 can be used to detect B-cell or non-Hodgkin lymphoma (NHL), chronic lymphocytic leukemia (CLL), and Hodgkin disease (HD) ; granulocyte-monocyte colony-stimulating factor receptor can be used to detect acute myelogenous leukemia (AML); CD25 can be used in the detection of CD25⁺ T- and B-cell malignancies and cells shed from solid tumors; CD27 can be used in the detection of in indolent NHL; CD40 can be used in multiple myeloma (MM), CLL, mantle cell lymphoma (MCL), and AML; CD44 can be used in NHL and early CLL; CD52 can be used in CLL; CD80 can be used in the detection of CLL and MCL; CD86 in MM and NHL; CD137 in leukemias and lymphomas; CD138 in MM and CLL; CD307 (IRTA2, FcRH5) can be used in the detection of hairy cell leukemia (HCL), MM, CLL, and MCL; beta₂-microglobulin in HD, MM, and cells shed from solid tumors; tumor necrosis factor receptor can be used in NHL; mesothelin can be used in the detection of mesotheliomas; CD22 can be used in HCL and insulin-like growth factor 1 receptor (IGFR1) can be used in the detection of prostate cancer. By way of illustration, a labeled Cys-Ser-Lys-Cys or Cys-Ser-Lys-Ala-Pro-Lys-Leu-Pro-Ala-Ala-Tyr-Cys (SEQ ID NO:8) peptide can be used to detect shed or circulating tumor that express IGFR1.

To the PACAP or VIP is coupled a label. A label in accordance with the present invention, is a fluorescent or luminescent molecule that can be detected using conventional microscopic techniques. A label of the invention can be or contain a chromophore, fluorophore or protein. In one embodiment, the fluorescent label may be green fluorescent protein (GFP) or a derivative thereof, e.g., yellow fluorescent protein (YFP), cyan fluorescent protein (CFP), Orange Fluorescent Protein (OFP), enhanced green fluorescent protein (eGFP), modified GFP (emGFP), enhanced yellow fluorescent protein (eYFP); monomeric red fluorescent protein (mRFP); mcherry. In another embodiment, the fluorescent label is fluorescein or rhodamine, as well as fluorescein or rhodamine derivatives, such as fluorescein isothiocyanate (FITC), OREGON Green, Tokyo Green, SNAFL, carboxynaphthofluorescein, ALEXA 488, DYLIGHT 488, tetramethylrhodamine (TAMRA) and its isothiocyanate derivative (TRITC), sulforhodamine 101, Rhodamine Red, ALEXA 546, ALEXA 555, ALEXA 633, DYLIGHT 549, DYLIGHT 633, 4a-diaza-s-indacene (BODIPY), CY-3, CY-5, CY-7, CY-CHROME, R-phycoerythrin (R-PE), etc. Luminescent labels include, but are not limited to, luciferase and aequorin.

In particular embodiments, the label is a near infrared (NIR) fluorophore. For the purposes of this invention, a near-infrared fluorophore includes dyes and other fluorophores with emission wavelengths (*e*.*g*., peak emission wavelengths) between about 680 and 1000 nm, *e.g.,* between about 680 and 800 nm, between about 800 and 900 nm, between 700 and 900 nM, between about 900 and 1000 nm, between about 680 and 750 nm, between about 750 and 800 nm, between about 800 and 850 nm, between about 850 and 900 nm, between about 900 and 950 nm, or between about 950 and 1000 nm. Fluorophores with emission wavelengths *(e.g.,* peak emission wavelengths) greater than 1000 nm can also be used in the methods described herein. Exemplary fluorophores include indocyanine green (ICG), IRDYE78, IRDYE80, IRDYE38, IRDYE40, IRDYE41, IRDYE700, IRDYE800, CY5.5, CY7, CY7.5, IR-786, DRAQ5NO (an N-oxide modified anthraquinone), quantum dots, and analogs thereof, e.g., hydrophilic analogs such as sulfonated analogs. Commercially obtainable fluorophores include dyes such as IRDYE78, and IRDYE800CW (LI-COR Biotechnology, Lincoln, NE), ALEXA Fluors 680, 700, and 750 (Invitrogen, Carlsbad, CA), and CYDYE Fluor CY5.5, CY7, AND CY7.5 (GE Healthcare, Chalfont St. Giles, UK). Ideally the fluorochromes are designed to emit at 800±100 nm.

As described herein, the PACAP can be assembled by standard Fmoc chemistry and coupled to the label via conventional chemistries. Likewise, VIP can be synthesized and labeled via conventional chemistries. In one embodiment, the label is coupled to a C-terminal residue of the PACAP or VIP. In another embodiment, the label is coupled to an N-terminal residue of the PACAP or VIP. In a further embodiment, the label is coupled to an amino acid side chain (*e*.*g*., lysine). For example, amine-reactive labels (*e*.*g*., fluorescent dyes) can be used to modify peptides at the N-terminal or a lysine residue. Three major classes of amine-reactive fluorescent reagents can used to label peptides: succinimidyl esters (SE), isothiocyanates and sulfonyl chlorides.

In addition, amine-containing labels can be used to modify peptides using water-soluble carbodiimides (such as 1-ethyl-3-(3-dimethylaminopropyl)carbodiimide (EDC)) to convert the carboxy groups of the peptides into amide groups. N-hydroxysuccinimide (NHS) may be used to improve the coupling efficiency of EDC-mediated protein-carboxylic acid conjugations. A large excess of the amine-containing labels is usually used for EDC-mediated bioconjugations in concentrated peptide solutions at low pH to reduce intra- and inter-protein coupling to lysine residues.

Because free thiol (SH) groups are not present as abundantly as amino groups in most peptides, thiol-reactive reagents often provide a means of selectively modifying a peptide at a defined site. Therefore thiol-reactive labels can be used to prepare labeled peptides. There are many types of thiol-reactive labels reported in the literature, including iodoacetamides, disulfides, maleimides, vinyl sulfones and various electron-deficient aryl halides and sulfonates. In particular embodiments, the label is functionalized with an iodoacetamide and maleimide moiety. Examples of such labels include, but are not limited to NIR FLUOR780 maleimide, TIDE FLUOR 5 maleimide, and NIR-830 maleimide.

Using the labeled PACAP or VIP described herein, shed or circulating tumor cells from prostate cancer can be readily detected in a urine sample. As used herein, shed tumor cells are defined as tumor cells that are released or shed from a primary tumor and transported through the circulatory system to distant sites. The term circulating tumor cells is used herein to mean cells of the blood that are cancerous.

For the purposes of this invention, a urine sample is intended to mean a urine sample obtained from a subject with prostate cancer, a subject suspected of having prostate cancer (*e*.*g*., exhibiting one or more symptoms associated with prostate cancer), a subject at risk of having prostate cancer (*e*.*g*., because of predisposition or exposure to a prostate carcinogen) or a subject who has been treated for prostate cancer. Desirably, the urine sample is obtained from a mammal, such as a canine, feline, rodent (*e*.*g*., mouse and rat), bovine, ovine, or and primate (*e.g.,* human). In a particular embodiment, the urine sample is obtained from a human.

Upon contact of a urine sample fluid with the labeled PACAP or VIP, PACAP binds to VPAC receptors present on the surface of shed or circulating tumor cells from prostate cancer, and binding to the shed or circulating tumor cells from prostate cancer is detected. In some embodiments, the cells in the urine sample are concentrated *(e.g.,* by centrifugation or filtration) prior to being contacted with the labeled PACAP or VIP. Binding of the labeled PACAP or VIP to shed or circulating tumor cells from prostate cancer can be determined by conventional methods including but not limited to fluorescence microscopy, fluorescence microplate reader, flow cytometry, fluorometry, and absorption spectroscopy. Detection may also be by means of an image analysis system utilizing a video camera interfaced to a digitizer or other image acquisition system. Detection may also be by visualization through a filter as under a fluorescence microscope. The microscope may just provide a signal that is visualized by the operator. However, the signal may be recorded on photographic film or using a video analysis system. The signal may also simply be quantified in real-time using either an image analysis system or simply a photometer. In a particular embodiment, binding of the labeled PACAP or VIP to shed or circulating tumor cells from prostate cancer is via fluorescent microscopy.

The detection of shed or circulating tumor cells from prostate cancer in a urine sample is of use in disease screening, diagnosing disease, monitoring for recurrence or progression of disease, and/or determining effectiveness of therapeutic intervention (chemical or surgical). Therefore, the present invention also provides a method for diagnosing prostate cancer by contacting a urine sample from a subject with a labeled PACAP or VIP and determining whether shed or circulating tumor cells from prostate cancer are present in the urine sample. Using this method of the invention, shed tumor cells from prostate cancer can be detected in urine samples.

In some embodiments, the diagnostic method of this invention is of use in detecting early stage cancer. As used herein, the phrase "early stage cancer" refers to those cancers, which may have tumors too small to be detected by conventional methods such as tumors having a size of approximately 2X10⁸ cells or smaller.

Once the urine sample from a subject has been contacted with the labeled PACAP or VIP, binding of the labeled PACAP or VIP to cancer cells from prostate cancer in the urine sample is detected, *e*.*g*.*,* as described above. The presence or absence of binding of the labeled PACAP or VIP to cancer cells from prostate cancer can be assessed by comparing said binding in the test sample to labeled PACAP or VIP binding in a control sample, e.g., a biological fluid known to have shed or circulating tumor cells and/or a biological fluid free of shed or circulating tumor cells. The presence of binding of the labeled PACAP or VIP to shed or circulating tumor cells from prostate cancer in the urine sample is indicative of the subject having cancer. Thus, a diagnosis of cancer or recurrence of cancer can be made.

The invention will be further described in the following examples.

### Example 1: Confocal Imaging of Prostate Cancer Cells in Urine Samples

The PACAP peptide, His-Ser-Asp-Gly-Ile-Phe-Thr-Asp-Ser-Tyr-Ser-Arg-Tyr-Arg-Lys-Gln-Met-Ala-Val-Lys-Lys-Tyr-Leu-Ala-Ala-Val-Leu-γAba-Lys(R) (SEQ ID NO:3), was synthesized on a Wang resin using an ABI 341A peptide synthesizer (Applied Biosystems, Foster City, CA). The 27-amino acid long PACAP sequence was then assembled by standard Fmoc chemistry and coupled with a free sulfhydryl group attached to the C-terminus. This C-terminal sulfhydryl group was then reacted with a maleimide functionalized NIR dye to yield TP4303.

Urine samples from two patients, one with early phase prostate cancer and the other with advanced prostate cancer, were cytospinned, and cells were collected on glass slides. The cells were incubated with TP4303, washed with phosphate-buffered saline (PBS) and the slide was dried. The cells were treated with DAPI (4', 6-diamidino-2-phenylindole, a fluorescent stain that binds strongly to A-T rich regions in cellular DNA), a cover slip was placed on the sample and confocal microscopic analysis was performed (excitation, 730 µm; emission, 860 µm).

The results of this analysis showed that TP4303 bound to highly dense VPAC1 receptors expressed on the cell membrane of prostate cancer cells from both patients. In addition, while epithelial cells were present, as evidenced by DAPI staining, these cells did not express VPAC1 and did not interact with TP4303. Likewise, while sperm were identified in the sample, as evidenced by DAPI staining of the head, sperm do not express VPAC1 and did not interact with TP4303. Therefore, TP4303 could selectively identify only the cancerous cells and could distinguish the cancerous cells from the normal contaminating cells.

## Claims

1. A method for detecting shed or circulating tumor cells from prostate cancer comprising contacting a urine sample with a labeled pituitary adenylate cyclase activating peptide (PACAP) or vasoactive intestinal peptide (VIP), and determining binding of the PACAP or VIP to shed or circulating tumor cells from prostate cancer in the urine sample thereby detecting shed or circulating tumor cells from prostate cancer.

2. The method of claim 1, wherein the label that is coupled to the PACAP or VIP peptide is or contains a chromophore, fluorophore or protein.

3. A method for diagnosing prostate cancer comprising
(a) contacting a urine sample with a labeled pituitary adenylate cyclase activating peptide (PACAP) or vasoactive intestinal peptide (VIP);
(b) detecting binding of PACAP or VIP to shed or circulating tumor cells from prostate cancer in the urine sample;
(c) comparing binding of PACAP or VIP to shed or circulating tumor cells from prostate cancer in the urine sample to PACAP or VIP binding in a control sample; and
(d) diagnosing prostate cancer, wherein the presence of binding of PACAP or VIP to shed or circulating tumor cells from prostate cancer in the urine sample is indicative of the subject having cancer.

4. The method of claim 3, wherein the label that is coupled to the PACAP or VIP peptide is or contains a chromophore, fluorophore or protein.

## Patentansprüche

1. Verfahren zum Nachweis von ausgeschütteten oder zirkulierenden Tumorzellen von Prostatakrebs, umfassend das Inkontaktbringen einer Urinprobe mit einem markierten Hypophysenadenylatcyclase-aktivierenden Peptid (PACAP) oder vasoaktiven Darmpeptid (VIP) und Bestimmen der Bindung von PACAP oder VIP an ausgeschüttete oder zirkulierende Tumorzellen von Prostatakrebs in der Urinprobe, wodurch ausgeschüttete oder zirkulierende Tumorzellen von Prostatakrebs nachgewiesen werden.

2. Verfahren nach Anspruch 1, wobei der Marker, der an das PACAP- oder VIP-Peptid gekoppelt ist, ein Chromophor, Fluorophor oder Protein ist oder enthält.

3. Verfahren zur Diagnose von Prostatakrebs, umfassend
(a) Inkontaktbringen einer Urinprobe mit einem markierten Hypophysenadenylatcyclase-aktivierenden Peptid (PACAP) oder vasoaktiven Darmpeptid (VIP);
(b) Nachweis der Bindung von PACAP oder VIP an ausgeschüttete oder zirkulierende Tumorzellen von Prostatakrebs in der Urinprobe;
(c) Vergleichen der Bindung von PACAP oder VIP an ausgeschüttete oder zirkulierende Tumorzellen von Prostatakrebs in der Urinprobe mit der PACAP- oder VIP-Bindung in einer Kontrollprobe; und
(d) Diagnostizieren von Prostatakrebs, wobei das Vorhandensein einer Bindung von PACAP oder VIP an ausgeschüttete oder zirkulierende Tumorzellen von Prostatakrebs in der Urinprobe darauf hinweist, dass der Patient Krebs hat.

4. Verfahren nach Anspruch 3, wobei die Marker, der an das PACAP- oder VIP-Peptid gekoppelt ist, ein Chromophor, Fluorophor oder Protein ist oder enthält.

## Revendications

1. Procédé de détection de cellules tumorales excrétées ou circulantes provenant d'un cancer de la prostate comprenant la mise en contact d'un échantillon d'urine avec un peptide d'activation de l'adénylate cyclase hypophysaire marqué (PACAP) ou un peptide intestinal vasoactif (VIP), et la détermination de la liaison du PACAP ou du VIP à les cellules tumorales excrétées ou circulantes provenant d'un cancer de la prostate dans l'échantillon d'urine, détectant ainsi les cellules tumorales excrétées ou circulantes provenant du cancer de la prostate.

2. Procédé selon la revendication 1, dans lequel le marqueur qui est couplé au peptide PACAP ou VIP est ou contient un chromophore, un fluorophore ou une protéine.

3. Procédé de diagnostic du cancer de la prostate comprenant
(a) la mise en contact d'un échantillon d'urine avec un peptide d'activation de l'adénylate cyclase hypophysaire marqué (PACAP) ou un peptide intestinal vasoactif (VIP);
(b) la détection de la liaison de PACAP ou VIP à des cellules tumorales excrétées ou circulantes provenant d'un cancer de la prostate dans l'échantillon d'urine;
(c) comparer la liaison de PACAP ou de VIP à les cellules tumorales excrétées ou circulantes provenant d'un cancer de la prostate dans l'échantillon d'urine à la liaison de PACAP ou de VIP dans un échantillon témoin; et
(d) diagnostiquer le cancer de la prostate, dans lequel la présence de liaison de PACAP ou de VIP à les cellules tumorales excrétées ou circulantes provenant d'un cancer de la prostate dans l'échantillon d'urine indique que le sujet a un cancer.

4. Procédé selon la revendication 3, dans lequel le marqueur qui est couplé au peptide PACAP ou VIP est ou contient un chromophore, un fluorophore ou une protéine.


### SEQUENCE LISTING

<110> Thomas Jefferson University
   Thakur, Mathew L.
   Gomella, Leonard
<120> Method for Detecting Shed or Circulating Tumor Cells in Biological Fluids
<130> JFU0003WO
<150> US 61/765,329
   <151> 2013-02-15
<160> 8
<170> PatentIn version 3.5
<210> 1
   <211> 38
   <212> PRT
   <213> Artificial sequence
<220>
   <223> Synthetic peptide
<400> 1
   His Ser Asp Gly Ile Phe Thr Asp Ser Tyr Ser Arg Tyr Arg Lys Gln
   1               5                   10                  15
   Met Ala Val Lys Lys Tyr Leu Ala Ala Val Leu Gly Lys Arg Tyr Lys
               20                  25                  30
   Gln Arg Val Lys Asn Lys
           35
<210> 2
   <211> 27
   <212> PRT
   <213> Artificial sequence
<220>
   <223> Synthetic peptide
<400> 2
   His Ser Asp Gly Ile Phe Thr Asp Ser Tyr Ser Arg Tyr Arg Lys Gln
   1               5                   10                  15
   Met Ala Val Lys Lys Tyr Leu Ala Ala Val Leu
               20                  25
<210> 3
   <211> 28
   <212> PRT
   <213> Artificial sequence
<220>
   <223> Synthetic peptide
<400> 3
   His Ser Asp Ala Val Phe Thr Asp Asn Tyr Thr Arg Leu Arg Lys Gln
   1               5                   10                  15
   Met Ala Val Lys Lys Tyr Leu Asn Ser Ile Leu Asn
               20                  25
<210> 4
   <211> 28
   <212> PRT
   <213> Artificial sequence
<220>
   <223> Synthetic peptide
<220>
   <221> MOD_RES
   <222> (1)..(1)
   <223> N-acetyl histidine
<220>
   <221> MOD_RES
   <222> (17)..(17)
   <223> methionine oxide
<400> 4
   His Ser Asp Ala Val Phe Thr Asp Asn Tyr Thr Arg Leu Arg Lys Gln
   1               5                   10                  15
   Met Ala Val Lys Lys Tyr Leu Asn Ser Ile Leu Asn
               20                  25
<210> 5
   <211> 28
   <212> PRT
   <213> Artificial sequence
<220>
   <223> Synthetic peptide
<220>
   <221> MOD_RES
   <222> (1)..(1)
   <223> N-acetyl histidine
<400> 5
   His Ser Asp Ala Val Phe Thr Asp Asn Tyr Thr Arg Leu Arg Lys Gln
   1               5                   10                  15
   Met Ala Val Lys Lys Tyr Leu Asn Ser Val Leu Asn
                       20                  25
<210> 6
   <211> 28
   <212> PRT
   <213> Artificial sequence
<220>
   <223> Synthetic peptide
<220>
   <221> MOD_RES
   <222> (1)..(1)
   <223> N-acetyl histidine
<220>
   <221> MOD_RES
   <222> (17)..(17)
   <223> methionine oxide
<400> 6
   His Ser Asp Ala Val Phe Thr Asp Asn Tyr Thr Arg Leu Arg Lys Gln
   1               5                   10                  15
   Met Ala Val Lys Lys Tyr Leu Asn Ser Val Leu Asn
               20                  25
<210> 7
   <211> 29
   <212> PRT
   <213> Artificial sequence
<220>
   <223> Synthetic peptide
<220>
   <221> MOD_RES
   <222> (28)..(28)
   <223> 4Abu
<400> 7
   His Ser Asp Gly Ile Phe Thr Asp Ser Tyr Ser Arg Tyr Arg Lys Gln
   1               5                   10                  15
   Met Ala Val Lys Lys Tyr Leu Ala Ala Val Leu Glu Lys
               20                  25
<210> 8
   <211> 12
   <212> PRT
   <213> Artificial sequence
<220>
   <223> Synthetic peptide
<400> 8
   Cys Ser Lys Ala Pro Lys Leu Pro Ala Ala Tyr Cys
   1               5                   10